# EUROPEAN PATENT APPLICATION

(11) **EP 1 589 508 A2**
(43) Date of publication of application: **26.10.2005**
(21) Application number: 05008470.6
(22) Date of filing: 19.04.2005
(51) Int. Cl.: G08B 21/06

(54) **Sound emitting device for maintaining correct posture or avoiding the effects of drowsiness**

(30) Priority: 22.04.2004 US 564684 P
(71) Applicant: D.E.H. L.L.C., Vero Beach, FL. 32960 (US)
(72) Inventor: The designation of the inventor has not yet been filed
(74) Representative: Schaad, Balass, Menzl & Partner AG

(57) **Abstract**

A device for assisting a wearer of the device to maintain correct posture or avoid the effects of drowsiness. The device comprises a tubular member which may be attached to an article of clothing or the body of a wearer, and the tubular member houses a ball, or a hammer, which impacts the wall of the tubular member so as to emit an audible sound or ping whenever the device is tilted from is initial position, to thereby warn the wearer of the tilted condition and so that corrective action can be taken.

## Description

### BACKGROUND OF THE INVENTION

The present invention relates to a sound emitting device which is designed to be attached to an article of clothing or the body of a wearer, to assist in maintaining a correct posture. The device may also be used to detect and warn of the nodding of the head associated with drowsiness.

Numerous studies have discussed the importance of good posture to good health. For example, the brochure entitled Posture for a Healthy Back, published by the Cleveland Clinic in 2001, lists the following benefits from proper posture:
- Keeps bones and joints in the correct alignment so that muscles are being used properly.
- Helps decrease the abnormal wearing of joint surfaces.
- Decreases the stress on the ligaments holding the joints of the spine together.
- Prevents the spine from becoming fixed in abnormal positions.
- Prevents fatigue because muscles are being used more efficiently, allowing the body to use less energy.
- Prevents backache and muscular pain.
- Contributes to a good appearance.

It is also well recognized that many activities, such as operating machinery or driving an automobile, can become extremely dangerous when the operator or driver becomes overly tired and drowsy.

It is accordingly an object of the present invention to provide a simple device for assisting a person in the maintenance of good posture, by monitoring for both forward and rearward leaning of the body from a position of good posture and alerting a wearer of the device so that corrective positioning can be undertaken.

It is also an object of the invention to provide a device of the described type which can also be used to monitor for a forward or rearward tilt of the head which is associated with drowsiness, and which can signal the wearer that such nodding has occurred.

### SUMMARY OF THE INVENTION

The above and other objects and advantages of the invention are achieved by the provision of a sound emitting device which comprises a tubular member configured to permit it to be attached to an article of clothing or the body of a wearer. The tubular member houses an internal ball or hammer for impacting the wall of the tubular member so as to emit a sound whenever the tubular member is pivoted a predetermined angle in either direction from an initial orientation. The device may thus be attached to an article of clothing or the body of the wearer so as to be disposed in the initial orientation when the wearer is in a position of correct posture, and when the wearer tilts either forwardly or rearwardly from the position of correct posture a sound is emitted which alerts the wearer to take corrective action.

In one embodiment, the tubular member has opposite end walls, and a ball is mounted within the tube so that it is free to roll between the end walls, Thus, the device may be attached to an article of clothing or the body of the wearer so that the tubular member is disposed substantially horizontally when the wearer is in a position of correct posture, and when the wearer tilts either forwardly or rearwardly from the position of correct posture the ball rolls into contact with the lower one of the end walls to emit a sound which alerts the wearer to take corrective action.

In a second embodiment, the tubular member may have closed or open ends, and a hammer is pivotally mounted within the tubular member. The hammer and the side wall of the tubular member are composed of materials which result in an audible sound being emitted whenever the hammer pivots into contact with the wall of the tubular member. Also, a spring biasing member is provided for supporting the hammer in a neutral position which is spaced from the wall of the tubular member. Thus in use, the device may be attached to an article of clothing or to the body of the wearer in a predetermined orientation when the wearer of the device is in a position of correct posture, and when the wearer tilts either forwardly or rearwardly from the position of correct posture the hammer pivots into contact with the wall of the tubular member to emit a sound which alerts the wearer to take corrective action.

### BRIEF DESCRIPTION OF THE DRAWINGS

Some of the objects and advantages of the present invention having been stated, others will appear from the following detailed disclosure, when considered in conjunction with the attached drawings, in which
Fig. 1 is a perspective view, partly broken away, of a first embodiment of the sound emitting device of the present invention, shown attached in an operative position to a cap of a wearer;
Fig. 2 is an exploded view of the sound emitting device shown in Fig. 1, with the ends of the device being opened to permit the ball to be received therein;
Fig. 3 is a fragmentary perspective end view of the device shown in Fig. 1;
Figs. 4A and 4B are schematic views showing the device of Fig. 1 in oppositely tilted orientations;
Fig. 5 is a schematic perspective view, partly broken away, showing a second embodiment of the device of the invention mounted within a cap of a wearer;
Fig. 6A is a fragmentary view of the device and cap of Fig. 5 in a position when the head of the wearer is tilted rearwardly,
Fig. 6B is a view similar to Fig. 6A showing the device and cap of Fig. 5 in a position when the head of the wearer is tilted forwardly;
Fig. 7 is a view similar to the upper portion of Fig. 1, and showing a another embodiment of the device;
Fig. 8 is a view similar to Fig. 7, and showing still another embodiment of the device;
Fig. 9 is a sectioned side elevation view of a further embodiment of a device which embodies the present invention; and
Fig. 10 is a sectional view taken along the line 10-10 of Fig. 9.

### DETAILED DESCRIPTION OF THE PREFERRED EMBODIMENTS

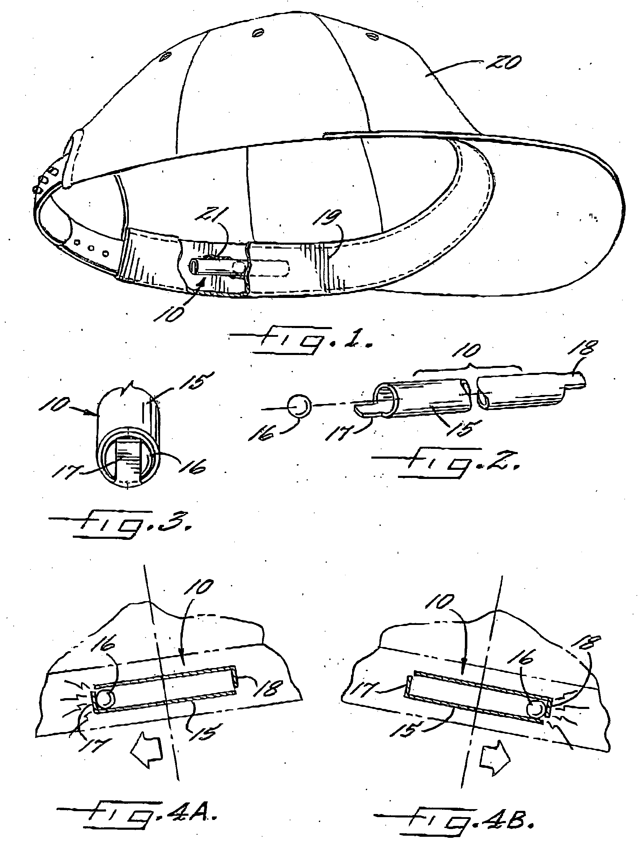

Figs. 1-3, 4A, and 4B illustrate a first embodiment of the invention, which comprises a sound emitting device **10** which comprises a cylindrical tube **15** which encloses a freely moveable ball **16**. The ball **1** may be composed of glass, metal, ceramic, or any other hard material which permits it to function in the manner described below.

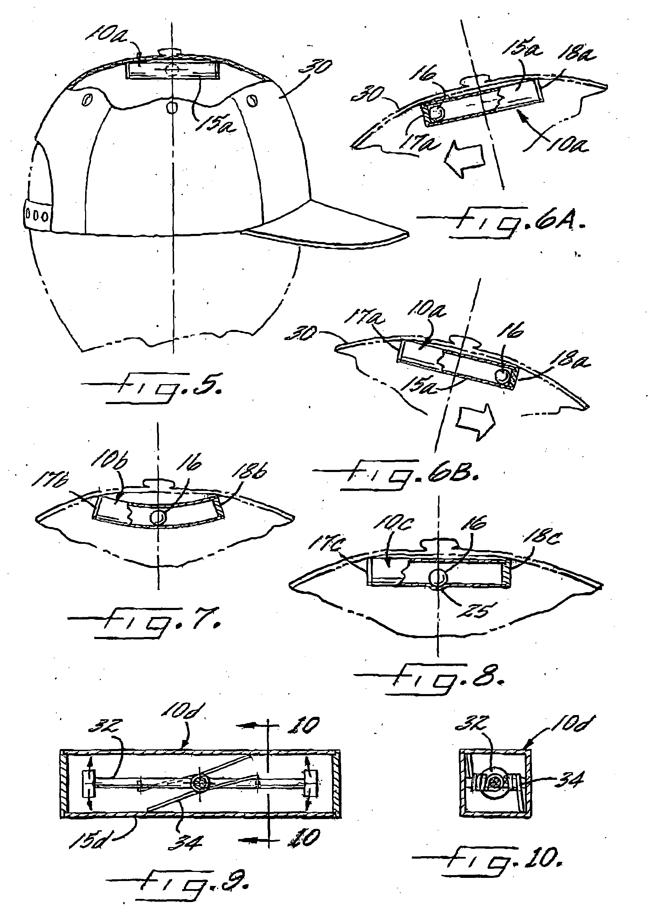

The tube **15** is preferably formed of a suitable metallic material, such as brass, and it includes opposite ends which are formed by integral end flaps **17** and **18.** The flaps **17** and **18** are initially formed in an open configuration as shown in Fig. 2 to permit assembly of the ball **16** within the tube, and the flaps are then folded upwardly to enclose the ball as seen in Figs. 1, 3, 4A, and 4B.

The sound emitting device **10** is configured so that it can be attached to an article of clothing or the body of the wearer, so that the tube **15** is disposed substantially horizontally when the wearer is in a position of correct posture. Thus, for example, and as shown in Fig. **1,** the device **10** may be attached within the band **19** of a cap **20**. A suitable adhesive **21** may be used to hold the device in the correct orientation within the band **19.** Also, by mounting the device within the band of the cap, it will be possible to locate the device immediately adjacent the ear of the wearer of the cap.

When the tube **15** is horizontal as seen in Fig. 1, the ball remains stationary, and when the level becomes inclined to a predetermined degree from horizontal in either direction, and as seen in Figs. 4A and 4B, the ball rolls into contact with the lowermost end flap **17** or **18** so as to emit a soft sound or ping.

Viewing Fig. 3, it will be seen that the end flaps **17** and **18** do not completely close the ends of the tube **15,** and thus the air which is in front the ball **16** as it moves along the tube is free to escape. Thus movement of the ball is not retarded by a closed air pocket in front of the ball.

Figs. 5, 6A, 6B, 7, and 8 illustrate further embodiments of the invention. Specifically, and as shown Fig 5, the device **10a** is mounted within the upper portion of a cap **30** so that the device is in a horizontal orientation when the wearer is in a position of correct posture. Also, as seen in Figs. 5, 6A, and 6B, the device **10a** has ends **17a, 18a** formed by separate members which close the ends of the tube **15a**. The tube **15a** and the ends **17a, 18a** are preferably formed of a metal, which functions to emit a sound when the ends are struck by the ball **16,** although other materials such as a hard plastic may be used. Also, it may be desirable to form a vent hole (not shown) through the ends **17a, 18a** to facilitate the release of the air in front of the moving ball.

It will be understood that when the wearer of the cap **20** or **30** assumes correct posture, the ball **16** may be disposed adjacent one of the ends of the tube **10** or **10a.** Thus when the wearer tilts in a direction to lower such one end, the ball may not strike such one end and no sound will be emitted. However, this occasional failure of the device to emit a sound has not been found to adversely effect the overall efficacy of the device.

In the embodiments of Figs. 7 and 8**,** the tube **10b** or **10c** is configured to releasably retain the ball **16** at a medial location along the length of the tube when the tube is in a horizontal orientation. For example, as illustrated in Fig. 7, the tube **10b** is formed with a shallow U-shaped curvature, so that the ball **16** is retained at the bottom of the U by gravity when the tube is horizontal. Alternatively, and as shown in Fig. 8, the ball **16** is releasably held in a small depression **25** at the medial location of the tube **10c,** in which case the tube could otherwise be a straight cylinder as in the case of the tubes **10** and **10a.** Thus in the embodiments of Figs. 7 and 8, the ball **16** will be assured to strike the lowered end of the tube and emit a sound whenever the wearer tilts from the correct posture in either direction.

As described above, the invention finds particular utility when the sound emitting device is attached to a hat, cap, or other headwear. For this purpose, the device **10a, 10b,** or **10c** may include an integral safety pin or the like (not shown), The tube of the device is oriented to be horizontal when the wearer's posture is properly upright, and the wearer is thereby signaled whenever his or her posture departs from a desired upright position, thus informing the wearer to correct his or her posture. It is believed that after extended use, the senses of the human body will react to the sound almost automatically to maintain a correct posture.

The invention also provides a unique function when the sound emitting device is worn while the wearer is jogging. More particularly, when the wearer jogs while in a proper upright position, the ball will bounce against the side wall in the tube and emit a continuous series of discrete sounds. However, if the wearer should lean either forward or backward to an unacceptable degree while jogging, the ball will move to one end of the tube and not emit the same continuous discrete sounds. Thus when the sounds cease, the wearer is notified that his or her posture should be corrected.

Figs. 9 and 10 illustrate a further embodiment wherein the sound emitting device comprises a hollow tube **15d** having either open or closed ends, and with a single or double ended hammer **32** pivotably mounted at a central location in the tube. When the tube is horizontal, the hammer is held in a neutral position by a spring biasing member **34**, and when the tube is moved in either direction from the horizontal, the hammer overcomes the spring member **34** and moves relative to the wall of the tube by reason of the inertia of the hammer, so that the hammer strikes the wall of the tube and emits a sound. Rather than using a spring biasing member, the hammer could be biased toward its neutral position by a suitable magnetic arrangement.

The embodiment of Figs. 9 and 10 can also be mounted in an orientation other than horizontal. For example, the tube **15d** could be attached to a hat, cap or other headwear in a vertical orientation, with the hammer **32** striking the inside wall of the tube whenever the tube is displaced from the vertical.

A second use for the device of the invention is to assist in avoiding the effects of drowsiness. When the device is attached to the hat or cap of an operator of machinery, or a driver of an automobile, any nodding of the head resulting from drowsiness will result in a sound being emitted which will alert the operator or driver in time to permit corrective action.

Many modifications and other embodiments of the invention set forth herein will come to mind to one skilled in the art to which the invention pertains, having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A device for assisting a wearer of the device to maintain a correct posture or avoid the effects of drowsiness, comprising
a tubular member configured to permit it to be attached to an article of clothing or the body of a wearer,
means mounted within the tubular member for impacting the wall of the tubular member so as to emit a sound whenever the tubular member is pivoted in either direction from an initial orientation,
whereby the device may be attached to an article of clothing or the body of the wearer so as to be disposed in said initial orientation when the wearer is in a position of correct posture, and when the wearer tilts from the position of correct posture a warning sound is emitted.

2. A device for assisting a wearer of the device to maintain a correct posture or avoid the effects of drowsiness, comprising
a tubular member having opposite end walls,
a ball retained within the tubular member so that the ball is free to roll into contact with either one of the end walls of the tubular member, said ball and end walls being composed of materials which result in an audible sound being emitted whenever the ball rolls into contact with one of the end walls,
whereby the device may be attached to an article of clothing or the body of the wearer so that the tubular support member is disposed substantially horizontally when the wearer is in a position of correct posture, and when the wearer tilts from the position of correct posture the ball rolls into contact with the lower one of the end walls to emit a warning sound.

3. The device of Claim 2 further comprising means interacting between the ball and the tubular member for releasably supporting the ball at a medial location along the length of the tubular member when the tubular member is in a horizontal orientation.

4. The device of Claim 3 wherein the supporting means comprises an arcuate curvature of the tubular member so that when the tubular member is oriented substantially horizontally, the ball will be moved by gravity to the medial location.

5. The device of Claim 3 wherein the supporting means comprises a detent formed in the side wall of the tubular member for releasably supporting the ball at the medial location when the tubular member is oriented substantially horizontally.

6. A device for assisting a wearer of the device to maintain a correct posture or avoid the effects of drowsiness, comprising
a tubular member,
a hammer pivotally mounted within the tubular member so as to be adapted to engage opposite wall portions of the tubular member,
said hammer and the opposite wall portions of the tubular member being composed of materials which result in an audible sound being emitted whenever the hammer pivots into contact with one of the opposite wall portions of the tubular member,
means for biasing the hammer toward a neutral position which is spaced from the opposite wall portions of the tubular member,
whereby the device may be attached to an article of clothing or to the body of the wearer in a predetermined orientation when the wearer of the device is in a position of correct posture, and when the wearer tilts either forwardly or rearwardly from the position of correct posture the hammer pivots against the force of the biasing means into contact with one of the opposite wall portions of the tubular member to emit a warning sound which alerts the wearer.

7. The device of Claim 6 wherein the biasing means comprises a spring.

8. An article of clothing having provision for assisting a wearer maintain correct posture or avoid the effects of drowsiness, comprising
a body garment,
a sound emitting device comprising a tubular member having opposite end walls, a ball retained within the tubular member so that the ball is free to roll into contact with either one of the end walls of the tubular member, said ball and end walls being composed of materials which result in an audible sound being emitted whenever the ball rolls into contact with one of the end walls,
said sound emitting device being mounted to the body garment so as to be disposed substantially horizontally when the body garment is worn by a wearer and the wearer is in a position of correct posture, and so that when the wearer moves from the position of correct posture the ball rolls into contact with one of the end walls and emits a warning sound.

9. The article of clothing of claim 8 wherein the body garment is a hat or cap.

10. The article of clothing of claim 9 wherein the sound emitting device is mounted within the band of the hat or cap so as to be positioned immediately adjacent the ear of the wearer.
